# EUROPÄISCHE PATENTSCHRIFT

(11) **EP 3 029 013 B1**
(45) Veröffentlichungstag und Bekanntmachung des Hinweises auf die Patenterteilung: **13.06.2018**
(21) Anmeldenummer: 14196195.3
(22) Anmeldetag: 04.12.2014
(51) Int. Cl.: C07F 15/00, B01J 31/18, C07F 9/6574, C07C 45/50

(54) **Monophosphite mit Struktureinheit 4,4,5,5-Tetraphenyl-1,3,2-dioxaphospholan als Liganden für Hydroformylierungskatalysatoren**
Monophosphites with structural unit 4,4,5,5-Tetraphenyl-1,3,2-dioxaphospholan as ligands for hydroformylation catalysts
Monophosphites à unités de structure 4,4,5,5-Tétraphényl-1,3,2-dioxaphospholane en tant que ligands de catalyseurs d'hydroformylation

(43) Veröffentlichungstag der Anmeldung: 08.06.2016
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Hess, Dieter, 45770 Marl (DE); Fridag, Dirk, 45721 Haltern am See (DE); Geilen, Frank, 45721 Haltern am See (DE); Selent, Detlef, 18059 Rostock (DE); Börner, Armin, 18059 Rostock (DE)

(56) Entgegenhaltungen:
- WO-A1-2008/071508
- WO-A2-2007/109549
- DETLEF SELENT ET AL: "A New Diphosphite Promoting Highly Regioselective Rhodium-Catalyzed Hydroformylation", ORGANOMETALLICS, Bd. 30, Nr. 17, 12. September 2011 (2011-09-12), Seiten 4509-4514, XP055186800, ISSN: 0276-7333, DOI: 10.1021/om2000508

## Beschreibung

Die Erfindung betrifft Monophosphite, die ein Benzpinakol aufweisen. Des Weiteren deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P(III). Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

In WO 2009/146984 werden Bisphosphite beschrieben, welche ein oder zwei Benzpinakol-Einheiten aufweisen. Auf Seite 13/14 sind diese als Strukturen Ia, Ib und Ic dargestellt. Bei den beiden Strukturen Ia und Ib werden die beiden Flügel-Bausteine durch einen Zentralbaustein verbunden, welcher eine Biphenoleinheit aufweist. Diese Biphenoleinheit weist in direkter Nachbarschaft zu den Sauerstoffatomen, welche mit dem P-Atom verknüpft sind jeweils eine *tert*.-Butylgruppe auf (siehe auch "A New Diphosphite Promoting Highly Regioselective Rhodium-Catalyzed Hydroformylation" von Detlef Selent, Robert Franke, Christoph Kubis, Anke Spannenberg, Wolfgang Baumann, Burkard Kreidler und Armin Börner in Organometallics 2011, 30, 4509-4514.)

In WO 2012/041846 wird auf Seite 16 ein Bisphosphit beschrieben (Struktur VIII), welches strukturgleich zu der Verbindung la aus der WO 2009/146984 ist.

DETLEF SELENT ET AL, "A New Diphosphite Promoting Highly Regioselective Rhodium-Catalyzed Hydroformylation", ORGANOMETALLICS, (20110912), Vol. 30, Nr. 17, Seiten 4509 - 4514 beschreibt ein hoch regioselektives Verfahren zur Rh-katalysierten Hydroformylierung.

In der WO 2008/071508 A1 werden Bisphosphitliganden für die übergangsmetallkatalysierte Hydroformylierung beschrieben.

In der WO 2007/109549 A2 wird ein Hydroformylierungsverfahren beschrieben, bei welchem die Reaktionsflüssigkeit mit einem komprimierten Gas angereichert ist.

Der Nachteil von Bisphosphiten ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität bei der Hydroformylierung von längerkettigen Olefinen, die durch hohe Verweilzeiten reaktionstechnisch kompensiert werden muss, was häufig einen großtechnischen Prozess unrentabel macht.

Der Erfindung lag die Aufgabe zugrunde, Phosphite bereitzustellen, welche gegenüber den bekannten Bisphosphiten einfacher herzustellen sind. Des Weiteren sollen diese Phosphite gute Eigenschaften in der Hydroformylierung von längerkettigen Olefinen aufweisen. Werden diese Phosphite in einer Hydroformylierungsreaktion verwendet, so sollen hier gute Ausbeuten erzielt werden.

Die Aufgabe wird gelöst durch eine Verbindung nach Anspruch 1.

Verbindung, welche eine der allgemeinen Strukturen **I** bis **V** aufweist: 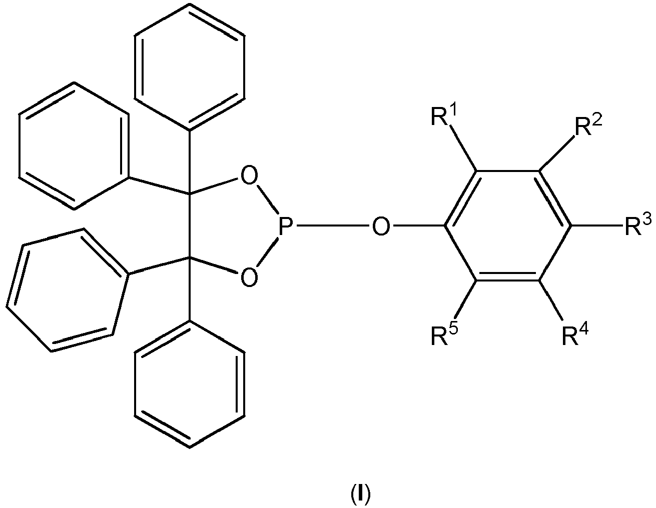 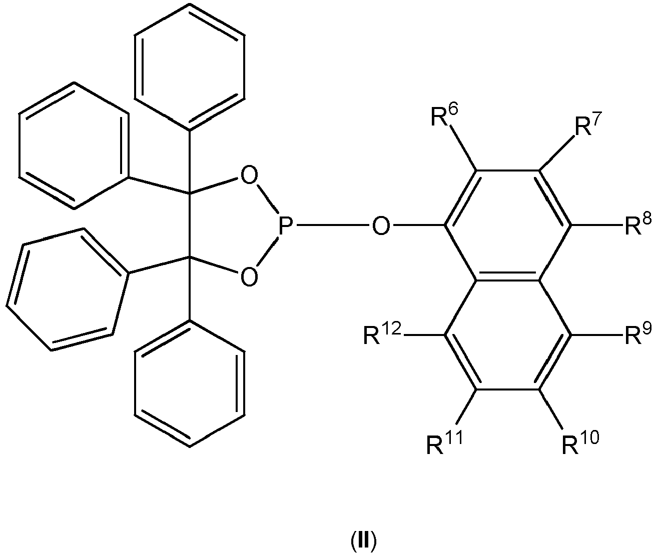 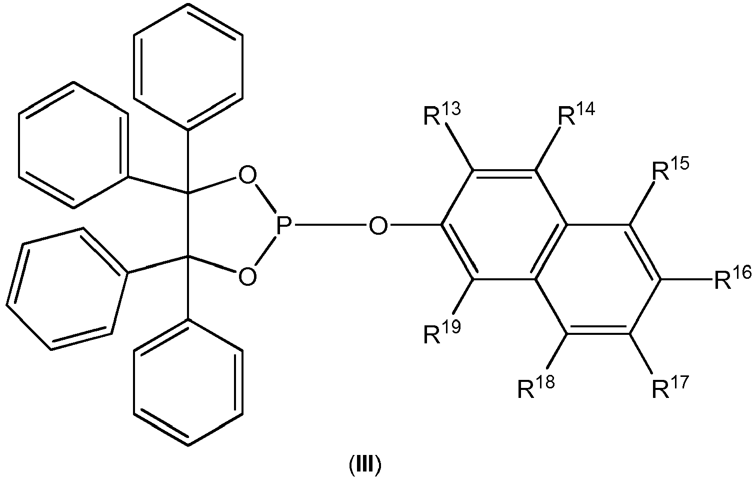 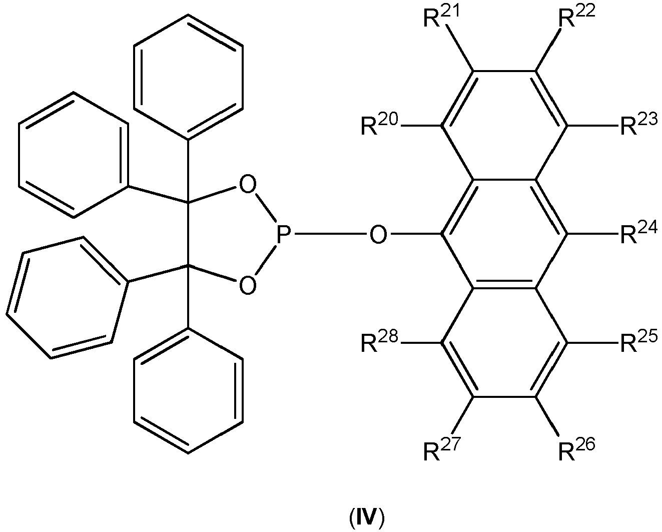 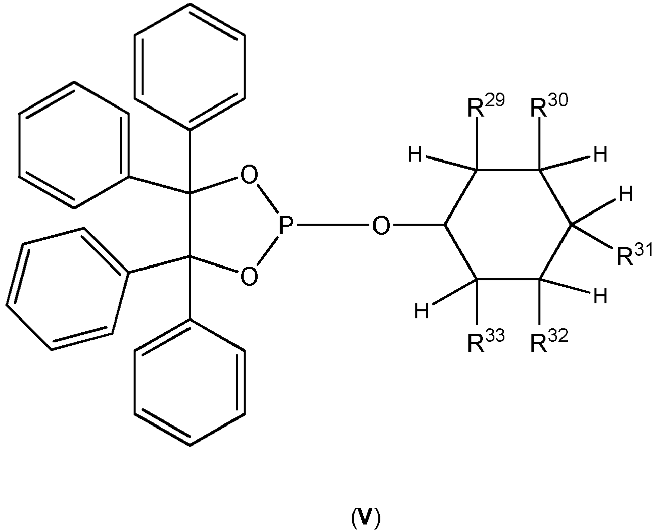 wobei
R¹, R², R³, R⁴, R⁵ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁶, R⁷, R⁸, R⁹, R¹⁰, R¹¹, R¹², D¹³, R¹⁴, R¹⁵, R¹⁶, R¹⁷, R¹⁸, R¹⁹, R²⁰, R²¹ R²², R²³, R²⁴, R²⁵, R²³, R²⁷, R²⁸ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
R²⁹, R³⁰, R³¹, R³², R³³ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl,-CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen folgendermaßen substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl:
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂;
und R¹ und R⁵ nicht für tert.-Butyl stehen,
und mindestens einer der Reste R¹, R², R³, R⁴, R⁵ nicht für -H steht,
und für den Fall, dass einer der Reste R¹, R², R³, R⁴, R⁵ für Phenyl steht, mindestens einer der vier verbleibenden Reste nicht für-H steht.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter-H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte-(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R⁶, R⁷, R⁸, R⁹, R¹⁰, R¹¹, R¹² ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R¹³, R¹⁴, R¹⁵, R¹⁶, R¹⁷, R¹⁸, R¹⁹ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R²⁹, R³⁰, R³¹, R³², R³³ ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R¹, R², R³, R⁴, R⁵ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R⁶, R⁷, R⁸, R⁹, R¹⁰, R¹¹, R¹² ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R¹³, R¹⁴, R¹⁵, R¹⁶, R¹⁷, R¹⁸, R¹⁹ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R²⁹, R³⁰, R³¹, R³², R³³ ausgewählt sind aus:
-H, -(C₁-C₁₂)-Alkyl, -O-(C₁-C₁₂)-Alkyl, -O-(C₆-C₂₀)-Aryl.

In einer Ausführungsform sind R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R¹, R², R³, R⁴, R⁵ ausgewählt sind aus:
-H, -(C₁-C₁₂)-Alkyl, -O-(C₁-C₁₂)-Alkyl, -O-(C₆-C₂₀)-Aryl.

In einer Ausführungsform sind R⁶, R⁷, R⁸, R⁹, R¹⁰, R¹¹, R¹² ausgewählt sind aus:
-H, -(C₁-C₁₂)-Alkyl, -O-(C₁-C₁₂)-Alkyl, -O-(C₆-C₂₀)-Aryl.

In einer Ausführungsform sind R¹³, R¹⁴, R¹⁵, R¹⁶, R¹⁷, R¹⁸, R¹⁹ ausgewählt sind aus:
-H, -(C₁-C₁₂)-Alkyl, -O-(C₁-C₁₂)-Alkyl, -O-(C₆-C₂₀)-Aryl.

In einer Ausführungsform sind R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl.

In einer Ausführungsform sind R²⁹, R³⁰, R³¹, R³², R³³ ausgewählt sind aus:
-H, -(C₁-C₁₂)-Alkyl, -O-(C₁-C₁₂)-Alkyl.

In einer Ausführungsform sind R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl.

In einer Ausführungsform sind R¹, R², R³, R⁴, R⁵ ausgewählt sind aus:
-H, -(C₁-C₁₂)-Alkyl, -O-(C₁-C₁₂)-Alkyl.

In einer Ausführungsform sind R⁶, R⁷, R⁸, R⁹, R¹⁰, R¹¹, R¹² ausgewählt sind aus:
-H, -(C₁-C₁₂)-Alkyl, -O-(C₁-C₁₂)-Alkyl.

In einer Ausführungsform sind R¹³, R¹⁴, R¹⁵, R¹⁶, R¹⁷, R¹⁸, R¹⁹ ausgewählt sind aus:
-H, -(C₁-C₁₂)-Alkyl, -O-(C₁-C₁₂)-Alkyl.

In einer Ausführungsform sind R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl.

In einer Ausführungsform sind R²⁹, R³⁰, R³¹, R³², R³³ ausgewählt sind aus:
-H, -(C₁-C₁₂)-Alkyl.

In einer Ausführungsform sind R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl.

In einer Ausführungsform weist die Verbindung die allgemeinen Strukturen **I** auf.

In einer Ausführungsform weist die Verbindung die allgemeinen Strukturen **II** auf.

In einer Ausführungsform weist die Verbindung die allgemeinen Strukturen **III** auf.

In einer Ausführungsform weist die Verbindung die allgemeinen Strukturen **IV** auf.

In einer Ausführungsform weist die Verbindung die allgemeinen Strukturen **V** auf.

In einer Ausführungsform weist die Verbindung eine der folgenden Strukturen (**1**) bis (**7**) auf:

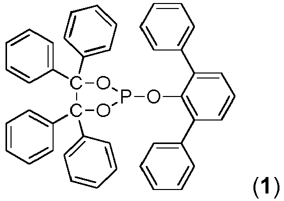

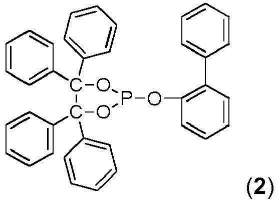

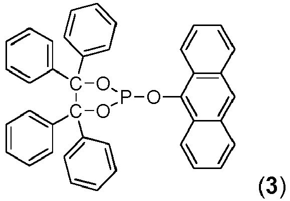

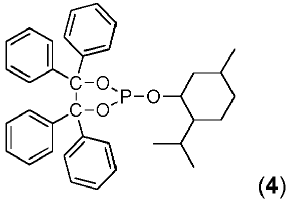

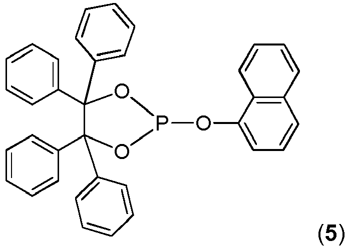

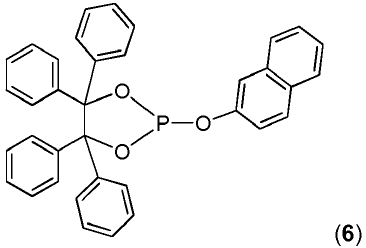

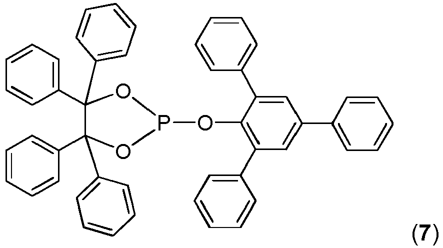

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar.

Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Biphenole

Die Synthese der Biphenole erfolgt analog zu DE102013203865 und DE102013203867.

### Synthese von 2-Chloro-4,4,5,5-tetraphenyl-1,3,2-dioxaphospholan

Die Synthese von 2-Chloro-4,4,5,5-tetraphenyl-1,3,2-dioxaphospholan erfolgt wie in in WO 2009/146984 bzw. "A New Diphosphite Promoting Highly Regioselective Rhodium-Catalyzed Hydroformylation" von Detlef Selent, Robert Franke, Christoph Kubis, Anke Spannenberg, Wolfgang Baumann, Burkard Kreidler und Armin Börner in Organometallics 2011, 30, 4509-4514 beschrieben.

Eine Suspension von 1,878 g (5,12 mmol) Benzopinakol (Acros, 98 %) in 30 mL Tetrahydrofuran (Sigma-Aldrich, 99,9 %) wurde bei -40 °C unter Rühren tropfenweise mit einer 0,768 molaren Lösung von Phosphortrichlorid (Aldrich, 99 %) in Tetrahydrofuran (10,7 mL; 8,22 mmol) und anschließend tropfenweise mit einer Lösung von 1,56 g (15,4 mmol) Triethylamin (Aldrich, p.a.) in 4 mL Tetrahydrofuran versetzt, wobei sich ein voluminöser farbloser Niederschlag bildete. Man ließ das Gemisch auf Raumtemperatur erwärmen und rührte es für weitere 5 h. Die Reaktionsmischung wurde über eine G4-Fritte filtriert und das Filtrat bei 20 mbar 1,5 h bei 40 °C getrocknet. Der viskose Rückstand wurde in Toluol (15 mL) aufgenommen. Diese Lösung wurde über eine G4-Fritte filtriert, das Filtrat bei 20 mbar eingeengt und anschließend für 2 h bei 40 °C Badtemperatur bei 10⁻¹ mbar getrocknet. Es wurden 2,32 g (90 % der Theorie, berechnet als Toluol-Addukt) einer hochviskosen Flüssigkeit erhalten, die noch 0,8 Äquivalente Toluol enthält.
Analyse: ³¹P{1H}-NMR (C₆D₆) δ=173,44 ppm.

### Synthese der Monophosphite

**2-([1,1':3',1"-Terphenyl]-2'-yloxy)-4,4,5,5-tetraphenyl-1,3,2-dioxaphospholan.**

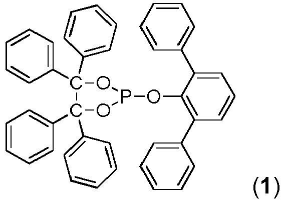

Eine Lösung von 2,6-Diphenylphenol (0,614 g; 2,493 mmol) in THF (5 ml) wurde mit einem Äquivalent n-Butyllithium, gelöst in Hexan (8 ml), bei -20 °C versetzt. Die Mischung wurde für 20 min bei -20 °C gerührt und auf Raumtemperatur erwärmt. Dann wurde eine Lösung von 2-Chloro-4,4,5,5-tetraphenyl-1,3,2-dioxaphospholan (1,074 g; 2,493 mmol) in THF (6 ml) tropfenweise zugegeben. Die Reaktionsmischung wurde über Nacht bei Raumtemperatur gerührt. Das Lösungsmittel wurde im Vakuum abgezogen, dann Toluol (15 ml) zugegeben und die resultierende Lösung zweimal filtriert. Das Filtrat wurde bis zur Trockne eingedampft. Der erhaltene hellgelbe Feststoff wurde für 4 h bei 50 °C getrocknet. Ausbeute: 1,448 g (2,260 mmol; 91 %).

Elementaranalyse (berechnet für C₄₄H₃₃O₃P = 640,72 g/ mol) C 82,32 (82,48); H 5,39 (5,19); P 4,94 (4,84) %.
³¹P-NMR (CD₂Cl₂): 144,7 ppm.
¹H-NMR (CD₂Cl₂): 87 (m, 4 H); 6,99 (m, 4 H); 7,08 (m, 2 H); 7,22-7,30 (m, 6 H); 7,30-7,37 (m, 9 H); 7,37-7,45 (m, 8 H) ppm.
¹³C-NMR (CD₂Cl₂): 94,9 (d, *J*_{CP}= 8,1 Hz); 125,0; 127,2; 127,3; 127,4; 127,5; 127,7; 128,6; 128,9; 129,0; 130,5; 130,5; 130,8; 136,6 (d, *J*_{CP}= 4,7 Hz); 138,5; 142,2 (d, *J*_{CP}= 4,6 Hz); 142,3; 147,0 (d, *J*_{CP}= 9,1 Hz) ppm.
ESI-TOF/HRMS: m/e 641,22372 (M+H)⁺.

### 2-([1,1'-Biphenyl]-2-yloxy)-4,4,5,5-tetraphenyl-1,3,2-dioxaphospholan.

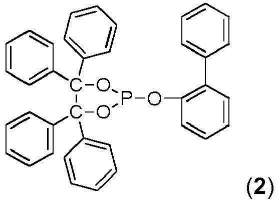

Eine Lösung von o-Phenylphenol (0,253 g; 1,487 mmol) in Toluol (3 ml) wurde mit Triethylamin (0,452 g; 4,463 mmol) versetzt. Die Mischung wurde auf 0 °C gekühlt und tropfenweise zu einer auf 0 °C gekühlten Lösung von 2-Chloro-4,4,5,5-tetraphenyl-1,3,2-dioxaphospholan (0,641 g; 1,488 mmol) in Toluol (5 ml) gegeben. Die Reaktionsmischung wurde über Nacht bei Raumtemperatur gerührt und dann filtriert. Das Filtrat wurde im Vakuum bis zur Trockne eingeengt. Ausbeute: 0,808 g (1,430 mmol; 96 %).

Elementaranalyse (berechnet für C₃₈H₂₉O₃P = 564,58 g/ mol) C 80,47 (80,83); H 5,07 (5,18); P 5,59 (5,49) %.
³¹P-NMR (CD₂Cl₂): 139,0 ppm.
¹H-NMR (CD₂Cl₂): 7,01-7,27 (m, 17 H); 7,27-7,32 (m, 2 H); 7,33-7,45 (m, 6 H); 7,54 (m, 4 H) ppm. ¹³C-NMR (CD₂Cl₂): 95,7 (d, *J*_{CP}= 8,4 Hz); 121,7 (d, *J*_{CP}= 12,9 Hz); 124,8; 127,5 (d, *J*_{CP}= 13,9 Hz); 127,6 (d, *J*_{CP}= 18,5 Hz); 128,4; 129,1 (d, *J*_{CP}= 4,3 Hz); 130,1; 130,3; 131,2; 134,2 (d, *J*_{CP}= 2,9 Hz); 140,0; 142,3 (d, *J*_{CP}= 4,7 Hz); 142,8; 148,8 (d, *J*_{CP}= 9,2 Hz) ppm.
ESI-TOF/HRMS: m/e 565,19253 (M+H)⁺.

### 2-(Anthracen-9-yloxy)-4,4,5,5-tetraphenyl-1,3,2-dioxaphospholan.

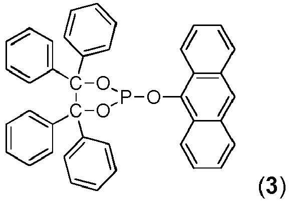

Eine gerührte Suspension von Anthracen-9-ol (0,252 g; 1,296 mmol) in THF (3 ml) wurde mit einem Äquivalent n-Butyllithium, gelöst in Hexan (4 ml), bei -20 °C versetzt. Die orange Mischung wurde für 20 min bei -20 °C gerührt und auf Raumtemperatur erwärmt. Dann wurde eine Lösung von 2-Chloro-4,4,5,5-tetraphenyl-1,3,2-dioxaphospholan (0,559 g; 1,296 mmol) in THF (3 ml) tropfenweise zugegeben. Die gelbe Reaktionsmischung wurde über Nacht bei Raumtemperatur gerührt und danach das Lösungsmittel im Vakuum entfernt. Toluol (10 ml) wurde zugegeben und die resultierende Lösung filtriert. Das Filtrat wurde bis zur Trockne eingedampft. Der erhaltene Feststoff wurde bei 50°C / 0,1 mbar getrocknet und durch Säulenchromatografie gereinigt (Hexan/Dichlormethan, 2:1, R*_{f}* = 0,5). Ausbeute: 0,554 g (0,942 mmol; 73 %).

Elementaranalyse (berechnet für C₄₀H₂₉O₃P = 588,64 g/ mol) C 81,56 (81,62); H 5,13 (4,97) %.
³¹P-NMR (CD₂Cl₂): 144,6 ppm.
¹H-NMR (CD₂Cl₂): 7,11-7,30 (m, 12 H); 7,30-7,41 (m, 7 H); 7,53 (m, 3 H); 7,91 (m, 3 H); 8,05 (m, 3 H); 8,31 (m, 1 H) ppm.
¹³C-NMR (CD₂Cl₂): 96,2 (d, *J*_{CP}= 8,1 Hz); 122,8; 123,2; 125,0 (d, *J*_{CP}= 3,9 Hz); 126,0 (d, *J*_{CP}= 6,0 Hz); 127,5; 127,6; 127,9; 128,1; 128,4; 128,5; 129,2 (d, *J*_{CP}= 3,4 Hz); 130,6; 132,5; 139,3; 142,2 (d, *J*_{CP}= 4,3 Hz); 143,0; 143,6 (d, *J*_{CP}= 7,2 Hz) ppm.
ESI-TOF/HRMS: m/e 589,19277 (M+H)⁺.

### 2-((2-Isopropyl-5-methylcyclohexyl)oxy)-4,4,5,5-tetraphenyl-1,3,2-dioxaphospholan.

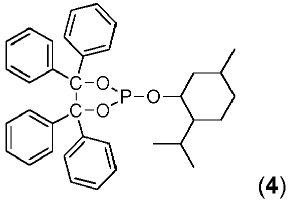

Eine Lösung von (-)-Menthol (0,225 g; 1,442 mmol) in THF (3 ml) wurde mit einem Äquivalent n-Butyllithium, gelöst in Hexan (4,5 ml), bei -20 °C versetzt. Die Mischung wurde für 20 min bei -20 °C gerührt und auf Raumtemperatur erwärmt. Dann wurde eine Lösung von 2-Chloro-4,4,5,5-tetraphenyl-1,3,2-dioxaphospholan (0,621 g; 1,442 mmol) in THF (4 ml) tropfenweise zugegeben, wobei ein Farbwechsel nach Blau, Grün und Gelb erfolgte. Die Reaktionsmischung wurde über Nacht gerührt und das Lösungsmittel im Vakuum entfernt. Toluol (10 ml) wurde zugegeben und die resultierende Lösung filtriert. Das Filtrat wurde bis zur Trockne eingeengt und der erhaltene Rückstand bei Raumtemperatur im Vakuum getrocknet. Ausbeute: 0,710 g (1,289 mmol; 89 %).

Elementaranalyse (berechnet für C₃₆H₃₉O₃P = 550,68 g/ mol) C 78,50 (78,52); H 7,23 (7,14); P 5,61 (5,62) %.
³¹P-NMR (CD₂Cl₂): 147,8 ppm.
¹H-NMR (CD₂Cl₂): 0,78 (m, 7 H); 0,89 (m, 3 H); 0,92-1,03 (m, 2 H); 1,27-1,51 (m, 2 H); 1,55-1,69 (m, 2 H); 1,69-1,78 (m, 1 H); 1,78-1,83 (m, 1 H); 3,83-3,98 (m, 1 H); 7,00-7,28 (m, 16 H, Hₐᵣₒₘ);
7,47-7,56 (m, 4 H, Hₐᵣₒₘ) ppm.
¹³C-NMR (CD₂Cl₂): 15,7; 21,1; 22,2; 23,1; 25,3; 26,0; 32,0; 34,5; 44,4; 48,7; 68,2; 75,0 (d, *J*_{CP}= 21,1 Hz); 94,5 (d, *J*_{CP}= 8,3 Hz); 94,7 (d, *J*_{CP}= 8,0 Hz); 127,2; 127,3; 127,4; 127,4; 129,1 (d, *J*_{CP}= 3,3 Hz); 129,2 (d, *J*_{CP}= 3,3 Hz); 130,4 (d, *J*_{CP}= 5,4 Hz); 142,9; 143,0; 143,2; 143,6 ppm.

### 2-(Naphthyl-1-oxy)-4,4,5,5-tetraphenyl-1,3,2-dioxaphospholan

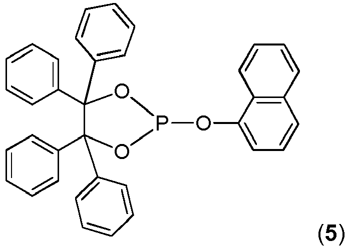

Eine Lösung von 1-Naphthol (0,231 g; 1,600 mmol) in THF (4 ml) wurde mit einem Äquivalent n-Butyllithium, gelöst in Hexan (5 ml), bei -20 °C versetzt. Die trübe Mischung wurde für 20 min bei -20 °C gerührt und auf Raumtemperatur erwärmt. Dann wurde eine Lösung von 2-Chloro-4,4,5,5-tetraphenyl-1,3,2-dioxaphospholan (0,689 g; 1,600 mmol) in THF (3 ml) tropfenweise zugegeben. Die Reaktionsmischung wurde über Nacht bei Raumtemperatur gerührt und danach das Lösungsmittel im Vakuum entfernt. Toluol (10 ml) wurde zugegeben und die resultierende Lösung filtriert. Das Filtrat wurde bis zur Trockne eingedampft. Der erhaltene Feststoff wurde für 5 h bei Raumtemperatur / 0,1 mbar getrocknet. Ausbeute: 0,660 g (1,227 mmol; 77 %).

Elementaranalyse (berechnet für C₃₆H₂₇O₃P = 538,16 g/ mol) C 80,62 (80,35); H 4,97 (4,98); P 5,74 (5,75) %.
³¹P-NMR (CD₂Cl₂): 139,3 ppm.
¹H-NMR (CD₂Cl₂): 7,09-7,26 (m, 11 H); 7,26-7,36 (m, 6 H); 7,37-7,60 (m, 4 H); 7,61-7,69 (m, 1 H); 7,69-7,81 (m, 4 H); 7,81-7,92 (m, 1 H) ppm.
¹³C-NMR (CD₂Cl₂): 95,8 (d, *J*_{CP} = 8 Hz), 115,1 (d, *J*_{CP} = 14 Hz), 122,8; 124,2; 126,0 (d, *J*_{CP} = 9 Hz); 126,9; 127,5; 127,7; 127,9; 128,6; 129,1 (d, *J*_{CP} = 3 Hz); 129,4; 130,4; 135,1; 142,3 (d, *J*_{CP} = 4 Hz); 143,0; 147,9 (d, *J*_{CP} = 9 Hz) ppm.
ESI-TOF/HRMS: m/e 577,13377 (M+K)⁺.

### 2-(Naphthyl-2-oxy)-4,4,5,5-tetraphenyl-1,3,2-dioxaphospholane

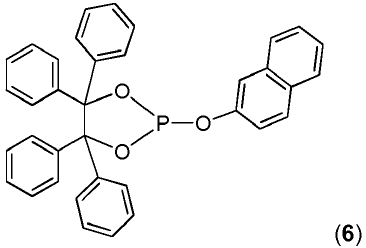

Eine Lösung von 2-Naphthol (0,231 g; 1,600 mmol) in THF (4 ml) wurde mit einem Äquivalent n-Butyllithium, gelöst in Hexan (5 ml), bei -20°C versetzt. Die gelbe Mischung wurde für 20 min bei -20 °C gerührt und auf Raumtemperatur erwärmt. Dann wurde eine Lösung von 2-Chloro-4,4,5,5-tetraphenyl-1,3,2-dioxaphospholan (0,689 g; 1,600 mmol) in THF (3 ml) tropfenweise zugegeben; nach 20 min trübte sich die Mischung. Die Reaktionsmischung wurde über Nacht bei Raumtemperatur gerührt und danach das Lösungsmittel im Vakuum entfernt. Toluol (10 ml) wurde zugegeben und die resultierende Lösung filtriert. Das Filtrat wurde bis zur Trockne eingedampft. Der erhaltene Feststoff wurde für 5 h bei Raumtemperatur / 0,1 mbar getrocknet. Ausbeute: 0,861 g (1,600 mmol; 100 %). Elementaranalyse (berechnet für C₃₆H₂₇O₃P = 538,16 g/ mol) C 80,57 (80,35); H 4,95 (4,98); P 5,77 (5,75) %.
³¹P-NMR (CD₂Cl₂): 138,5 ppm.
¹H-NMR (CD₂Cl₂): 6,88-7,00 (m, 1 H); 7,07-7,25 (m, 10 H); 7,25-7,39 (m, 7 H); 7,41-7,60 (m, 2 H); 7,61-7,74 (m, 4 H); 7,74-7,92 (m, 3 H) ppm.
¹³C-NMR (CD₂Cl₂): 95,6 (d, *J*_{CP} = 8 Hz), 116,6 (d, *J*_{CP} = 11 Hz), 121,6 (d, *J*_{CP} = 6 Hz) ; 125,2; 125,7; 126,9; 127,4; 127,6; 127,7; 127,8, 128,0; 128,6, 129,1 (d, *J*_{CP} = 3 Hz); 129,4; 130,0; 130,3; 130,8; 134,4; 142,3 (d, *J*_{CP} = 4 Hz); 143,0; 149,2 (d, *J*_{CP} = 8 Hz) ppm.
ESI-TOF/HRMS: m/e 577,13431 (M+K)⁺.

### 4,4,5,5-Tetraphenyl-2-((5'-phenyl-[1,1':3',1"-terphenyl]-4'-yl)oxy)-1,3,2-dioxaphospholan

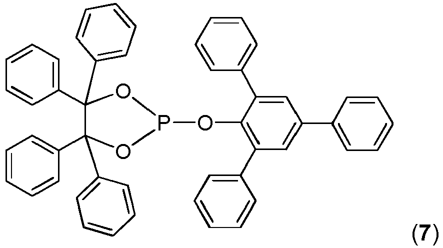

Zu einer auf -20 °C gekühlten Lösung von 2,4,6-Triphenylphenol (0,5 g; 1,55 mmol) in THF (12 ml) wird unter Rühren tropfenweise n-BuLi (1 ml einer 1,6 M Lösung in Hexan = 1,6 mmol) gegeben. Man lässt auf Raumtemperatur erwärmen und versetzt dann mit einer Suspension von 2-Chloro-4,4,5,5-tetraphenyl-1,3,2-dioxaphospholan (0,667 g; 1,55 mmol) in THF (6 ml). Es wird 24 h bei Raumtemperatur gerührt, das Lösungsmittel in Vakuum entfernt und Toluol (8 ml) zugesetzt. Es wird filtriert. Das Filtrat wird zur Trockne eingeengt und der Rückstand säulenchromatografisch mit Toluol als Laufmittel gereinigt. Ausbeute: 0,201 g (0,279 mmol; 18%).
³¹P-NMR (CD₂Cl₂): *δ* 144,1 ppm.
¹H-NMR (CD₂Cl₂): *δ* 7,67-6,81 (m, 37 H) ppm.
¹³C-NMR (75,5 MHz, CD₂Cl₂): *δ* = 149,5; 142,6; 142,2 (d, *J*_{CP} = 4,5); 140,9; 140,6; 138,5; 138,0; 137,8; 136,8; 136,8; 134,1; 130,8; 130,5; 129,8; 129,6; 129,4; 129,3; 129,2; 129,1; 128,9; 129,0; 128,6; 128,4; 128,1; 128,0; 127,7; 127,7; 127,4; 127,3; 127,2; 127,1; 125,6; 94,8 (d, *J*_{CP} = 8,7 Hz) ppm.

### 4,4,5,5-Tetraphenyl-2-(2,4,6-tri-tert-butylphenoxy)-1,3,2-dioxaphospholan.

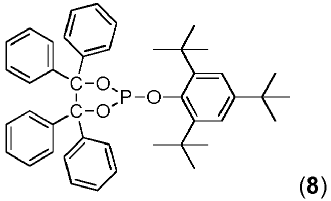

Eine Lösung von 2,4,6-Tri-*tert*-butylphenol (0,315 g; 1,201 mmol) in THF (3 ml) wurde mit einem Äquivalent n-Butyllithium, gelöst in in Hexan (4 ml), bei Raumtemperatur versetzt. Nach 10-minütigem Rühren wurde die erhaltene rosa-gelbe Suspension tropfenweise zu einer Lösung von 2-Chloro-4,4,5,5-tetraphenyl-1,3,2-dioxaphospholan (0,518 g; 1,203 mmol) in THF (3 ml) gegeben. Die Reaktionsmischung wurde für 10 min bei -20 °C und über Nacht bei Raumtemperatur gerührt. Das Lösungsmittel wurde im Vakuum entfernt, Toluol (15 ml) zugegeben und die resultierende Lösung filtriert. Das Filtrat wurde bis zur Trockne eingedampft. Der erhaltene Feststoff wurde für 1 h bei 40°C / 0,1 mbar getrocknet. Zuerst wurde aus heißem Hexan (26 ml) und dann aus Acetonitril (2 ml) umkristallisiert. Ausbeute: 0,205 g (0,312 mmol; 26 %).
³¹P-NMR (CD₂Cl₂): 145,8 ppm.
¹H-NMR (CD₂Cl₂): 1,42 (s, 9 H); 1,47 (s, 18 H); 7,06-7,33 (m, 16 H, Hₐᵣₒₘ); 7,45 (s, 2 H, Hₐᵣₒₘ); 7,71-7,76 (m, 4 H, Hₐᵣₒₘ) ppm.
¹³C-NMR (CD₂Cl₂): 31,7; 32,0; 32,0; 35,0; 35,7; 96,1 (d, *J*_{CP}= 9,3 Hz); 123,6; 127,4; 127,4; 127,7; 127,8; 129,1 (d, *J*_{CP}= 4,3 Hz); 130,7; 142,4 (d, *J*_{CP}= 4,8 Hz); 142,6; 143,1 (d, *J*_{CP}= 3,2 Hz); 145,9; 148,2 (d, *J*_{CP}= 11,8 Hz) ppm.
ESI-TOF/HRMS: m/e 657,34885 (M+H)⁺.

### 8-((4,4,5,5-Tetraphenyl-1,3,2-dioxaphospholan-2-yl)oxy)chinolin

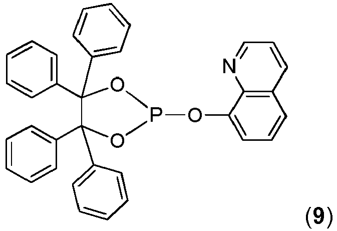

Eine Lösung von Chinolin-8-ol (0,232 g; 1,600 mmol) in THF (4 ml) wurde mit einem Äquivalent n-Butyllithium, gelöst in Hexan (5 ml), bei -20 °C versetzt. Die gelbe Mischung wurde für 20 min bei -20 °C gerührt und auf Raumtemperatur erwärmt. Dann wurde eine Lösung von 2-Chloro-4,4,5,5-tetraphenyl-1,3,2-dioxaphospholan (0,689 g; 1,600 mmol) in THF (3 ml) tropfenweise zugegeben, nach 15 min bildete sich Niederschlag. Die Reaktionsmischung wurde über Nacht bei Raumtemperatur gerührt und danach das Lösungsmittel im Vakuum entfernt. Toluol (10 ml) wurde zugegeben und die Suspension erhitzt, danach wurde heiß filtriert. Das Filtrat wurde bis zur Trockne eingedampft. Der erhaltene Feststoff wurde bei 50°C / 0,1 mbar getrocknet. Dann wurde die Substanz in Hexan (15 ml) verrührt und die Suspension filtriert. Der erhaltene Feststoff wurde für 4 h bei 50°C / 0,1 mbar getrocknet. Ausbeute: 0,760 g (1,409 mmol; 88 %).

Elementaranalyse (berechnet für C₃₅H₂₆O₃PN = 539,57 g/ mol) C 77,90 (77,91); H 4,96 (4,86); P 5,63 (5,74); N 2,47 (2,60) %.
³¹P-NMR (CD₂Cl₂): 134,8 ppm.
¹H-NMR (CD₂Cl₂) 74-6,77 (m, 1 H); 7,09-7,14 (m, 2 H); 7,14-7,26 (m, 14 H); 7,40 (t, 1 H); 7,49-7,56 (m, 2 H); 7,58-7,64 (m, 4 H); 8,21-8,25 (m, 1 H); 9,06-9,09 (m, 1 H) ppm.
¹³C-NMR (CD₂Cl₂): 95,3 (d, *J*_{CP} = 8 Hz), 118,4; 122,1; 122,2; 127,4; 127,5; 127,5; 129,3 (d, *J*_{CP} = 4 Hz); 130,2; 136,3; 142,9 (d, *J*_{CP} = 4 Hz); 143,6; 149,0; 149,8 ppm.
ESI-TOF/HRMS: m/e 540,17262 (M+H)⁺.

### 2-Phenoxy-4,4,5,5-tetraphenyl-1,3,2-dioxaphospholan

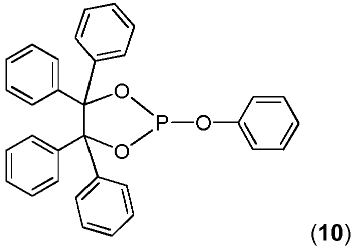

Zu einer gerührten Lösung von Phenol (0,186 g; 1,98 mmol) in Toluol (5 ml) wird bei Raumtemperatur Triethylamin (0,301 g; 2,97 mmol) und anschließend tropfenweise eine Lösung von 2-Chloro-4,4,5,5-tetraphenyl-1,3,2-dioxaphospholan (0,853 g; 1,98 mmol) in Toluol (5 ml) gegeben. Es wird über Nacht gerührt, filtriert, und das Filtrat eingeengt. Ausbeute Rohprodukt: 0,890 g. Das Rohprodukt wird nochmals in Toluol aufgenommen, und die erhaltene Lösung durch eine Schicht Kieselgel filtriert. Das Filtrat wird zur Trockne eingeengt, und bei 50°C/ 0,1 mbar getrocknet.

Elementaranalyse (berechnet für C₃₂H₂₅O₃P = 488,15 g/mol): C, 78,48 (78,68); 5,19 (5,16); P, 6,30 (6,34) %.
³¹P-NMR (121 MHz, CD₂Cl₂): *δ* 138,5 ppm.
¹H-NMR (CD₂Cl₂): *δ* 7,58-7,50 (m, 4H), 7,27-7,01 (m, 19H), 6,75-6,69 (m, 2H), 1,46 ppm.
¹³C-NMR (CD₂Cl₂): *δ* 151,5 (d, *J*_{CP} = 8,7 Hz), 143,0; 142,3 (d_{CP}, *J* = 4,2 Hz), 130,2; 129,9; 129,1 (d, *J*_{CP} = 3,3 Hz), 127,7 (d, *J*_{CP} = 16,7 Hz), 127,6; 127,4, 124,3, 121,0 (d, *J*_{CP} = 8,2 Hz), 95,5 (d, *J*_{CP} = 8,2 Hz) ppm.

### 2-([1,1'-Biphenyl]-3-yloxy)-4,4,5,5-tetraphenyl-1,3,2-dioxaphospholane

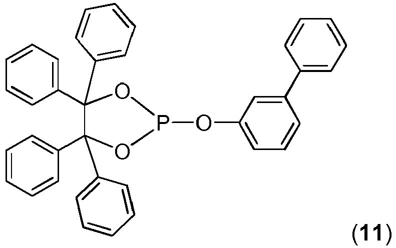

Zu einer gerührten Lösung von 3-Phenylphenol (0,373 g; 1,97 mmol) in Toluol (6 ml) wird bei Raumtemperatur Triethylamin (0,301 g; 2,97 mmol) und anschließend tropfenweise eine Lösung von 2-Chloro-4,4,5,5-tetraphenyl-1,3,2-dioxaphospholan (0,850 g; 1,97 mmol) in Toluol (6 ml) gegeben. Es wird über Nacht gerührt, filtriert, und das Filtrat im Vakuum zur Trockne eingeengt. Ausbeute: 1,07 g an Rohprodukt. Das Rohprodukt wird in Toluol aufgenommen und die erhaltene Lösung durch eine Schicht Kieselgel filtriert. Das Filtrat wird zur Trockne eingeengt, und bei 50°C/ 0,1 mbar getrocknet.

Elementaranalyse (berechnet für C₃₈H₂₉O₃P = 564,61 g/mol): C, 80,81 (80,84); 5,17 (5,18); P, 5,46 (5,49) %.
³¹P-NMR (CD₂Cl₂): *δ* 138,5 ppm.
¹H-NMR (CD₂Cl₂): *δ* 7,60-7,03 (m, 27H), 6,87-6,74 (m, 2H) ppm.
¹³C-NMR (CD₂Cl₂): *δ* 151,8 (d, *J*_{CP} = 8,1 Hz), 143,1 (d, *J*_{CP} = 5,2 Hz); 142,3 (d, *J*_{CP} = 4,1 Hz); 141,0 (d, *J*_{CP} = 5,0 Hz); 140,6; 130,4; 130,3; 130,2; 129,4; 129,3; 129,2, 129,1, 129,0, 128,7; 128,6; 128,5, 128,5; 128,4; 128,3; 128,0; 127,9; 127,8; 127,7; 127,6; 127,4; 127,2; 125,7; 123,0; 119,8; 119,7; 114,6; 114,3; 97,8; 95,6 (d, *J*_{CP} = 8,0 Hz) ppm.

### 2-([1,1'-Biphenyl]-4-yloxy)-4,4,5,5-tetraphenyl-1,3,2-dioxaphospholan

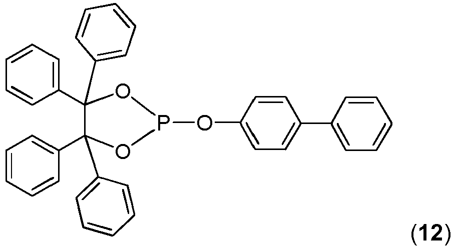

Zu einer gerührten Suspension von 4-Phenylphenol (0,337 g; 1,98 mmol) in Toluol (10 ml) wird bei Raumtemperatur Triethylamin (0,301 g; 2,97 mmol) und anschließend tropfenweise eine Lösung von 2-Chloro-4,4,5,5-tetraphenyl-1,3,2-dioxaphospholan (0,853 g; 1,98 mmol) in Toluol (5 ml) gegeben. Es wird über Nacht gerührt, filtriert, und das Filtrat nochmals durch eine dünne Schicht Kieselgel filtriert. Das Filtrat wird im Vakuum zur Trockne eingeengt. Ausbeute: 1,15 g. Das Rohprodukt wird erneut in Toluol (15 ml) aufgenommen und die erhaltene Lösung durch eine Schicht von Kieselgel filtriert. Einengen des Filtrates und Trocknen bei 0,1 mbar ergibt reines Monophosphit.

Elementaranalyse (berechnet für C₃₈H₂₉O₃P = 564,61 g/mol): C, 80,57 (80,84); 5,26 (5,18); P, 5,39 (5,49) %.
³¹P-NMR (CD₂Cl₂): *δ* 138,4 ppm.
¹H-NMR (CD₂Cl₂): *δ* 7,60-6,80 (m, 27H), 6,79 (m, 2H) ppm.
¹³C-NMR (CD₂Cl₂): *δ* 151,1 (d, *J*_{CP} = 8,4 Hz), 143,1; 142,3 (d, *J*_{CP}= 4,0 Hz), 140,8; 137,4; 130,3; 129,4; 129,1 (d, *J*_{CP} = 3,3 Hz), 128,6; 127,7 (d, *J*_{CP} = 21,0 Hz); 127,7; 127,5; 127,4, 127,2; 125,7; 121,2 (d, *J*_{CP} = 8,1 Hz); 95,6 (d, *J*_{CP} = 7,9 Hz) ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ∼3 %; *cis*+*trans*-2-Octen: ∼49%; *cis*+*trans*-3-Octen: ∼29%; *cis*+*trans*-Octen-4: ∼16%; gerüstisomere Octene: ∼3 % wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse, s.u.) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). Der Autoklav wurde bei einem Gesamtgasdruck (Synthesegas: Linde; H₂ (99,999%) : CO (99,997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

Als Vergleichsbeispiel wurde der in WO 2009/146984 bzw. "A New Diphosphite Promoting Highly Regioselective Rhodium-Catalyzed Hydroformylation" von Detlef Selent, Robert Franke, Christoph Kubis, Anke Spannenberg, Wolfgang Baumann, Burkard Kreidler und Armin Börner in Organometallics 2011, 30, 4509-4514 beschriebene Ligand (hier als Ligand A bezeichnet) verwendet.

### Vergleichsligand:

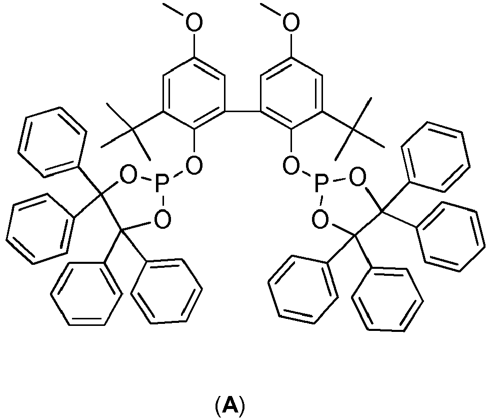

Die Herstellung des Liganden erfolgte nach den oben zitierten Vorschriften.

### Katalyseergebnisse

Die Ergebnisse der Katalyseversuche sind in den Tabellen 1 und 2 zusammengefasst.

**Tabelle 1:**

| **Ligand** | **p (bar)** | **T (°C)** | **t (h)** | **[Rh] (ppm)** | **P/Rh** | **Ausb. (%)** |
|---|---|---|---|---|---|---|
| **1*** | 50 | 120 | 4 | 100 | 5 | 97 |
| **2*** | 50 | 120 | 4 | 100 | 5 | 99 |
| **3*** | 50 | 110 | 4 | 100 | 5 | 97 |
| **3*** | 50 | 100 | 4 | 100 | 5 | 90 |
| **3*** | 50 | 80 | 4 | 40 | 5 | 92 |
| **4*** | 50 | 120 | 4 | 100 | 5 | 93 |
| **5*** | 50 | 110 | 4 | 100 | 5 | 98 |
| **6*** | 50 | 110 | 4 | 100 | 5 | 94 |
| **7*** | 50 | 120 | 4 | 100 | 5 | 89 |
| **8** | 50 | 120 | 4 | 100 | 5 | 18 |
| **9** | 50 | 120 | 4 | 100 | 5 | 2 |
| **10** | 50 | 120 | 4 | 100 | 5 | 40 |
| **11** | 50 | 120 | 4 | 100 | 5 | 9 |
| **12** | 50 | 120 | 4 | 100 | 5 | 3 |
| **A** | 50 | 120 | 4 | 100 | 5 | 7 |

| | | | | | | |
|---|---|---|---|---|---|---|
| * erfindungsgemäße Verbindung Olefin: n-Octene (Oxeno GmbH) Solvens: Toluol Verhältnis Phosphor/Rhodium (L/Rh): 5 : 1 | | | | | | |

Die in Tabelle 1 gelisteten Ergebnisse zeigen klar, dass der Einsatz der erfindungsgemäßen Verbindungen (**1**) bis (**7**) zu deutlich besseren Ausbeuten führt, als der Einsatz der Vergleichsverbindungen (**8**) bis (**12**). Ferne zeigt sich, dass die Ausbeuten der erfindungsgemäßen Monophosphite (**1**) bis (**7**) deutlich höher sind, als die Ausbeute des aus dem Stand der Technik bekannten Vergleichsliganden (**A**).

**Tabelle 2:**

| **Ligand** | **p (bar)** | **T (°C)** | **t (h)** | **[Rh] (ppm)** | **P/Rh** | **Ausb. (%)** |
|---|---|---|---|---|---|---|
| **3*** | 50 | 80 | 4 | 100 | 5 | 99 |
| **A** | 50 | 80 | 4 | 100 | 5 | 0,2 |

| | | | | | | |
|---|---|---|---|---|---|---|
| * erfindungsgemäße Verbindung Olefin: 2-Penten Solvens: Toluol Verhältnis Phosphor/Rhodium (L/Rh): 5 : 1 | | | | | | |

Die Verbindung (**3**) wurde nicht nur mit n-Octenen, sondern zusätzlich auch mit 2-Penten getestet. Auch hier konnte eine sehr gute Ausbeute erzielt werden. Mit Hilfe des Vergleichsliganden (**A**) konnten nur geringer Spuren an Zielprodukt generiert werden.

Anhand der oben beschriebenen Versuche konnte gezeigt werden, dass die gestellte Aufgabe durch die erfindungsgemäßen Verbindungen gelöst wird.

## Patentansprüche

1. Verbindung, welche eine der allgemeinen Strukturen **I** bis **V** aufweist: 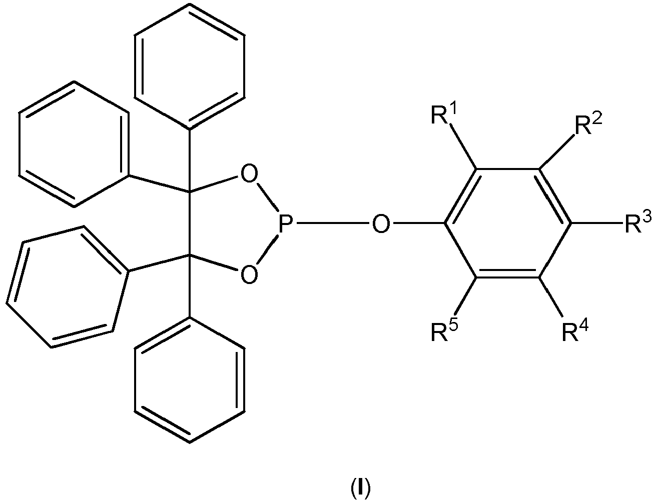 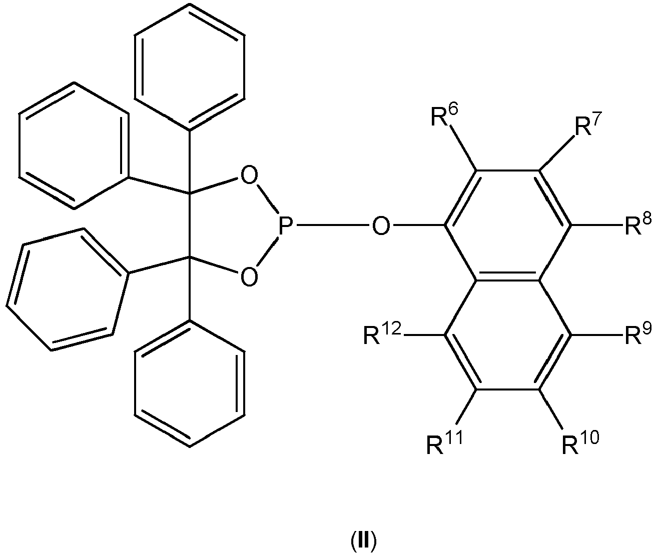 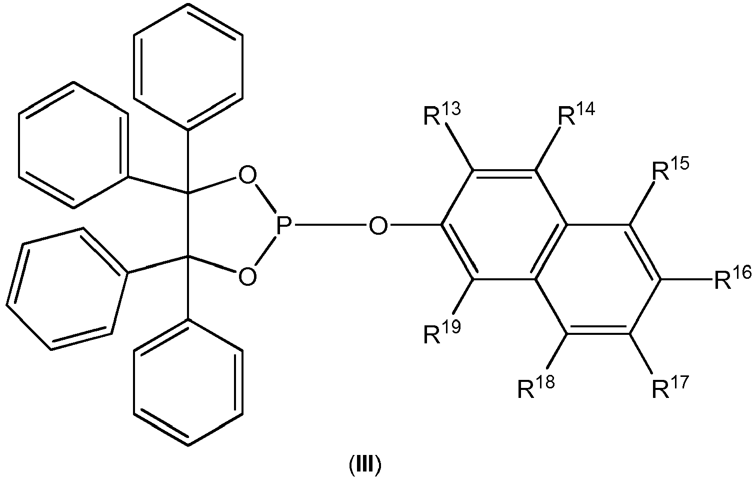 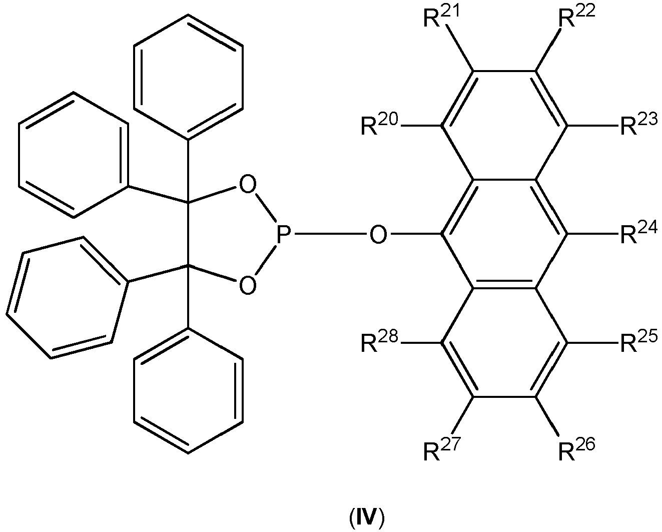 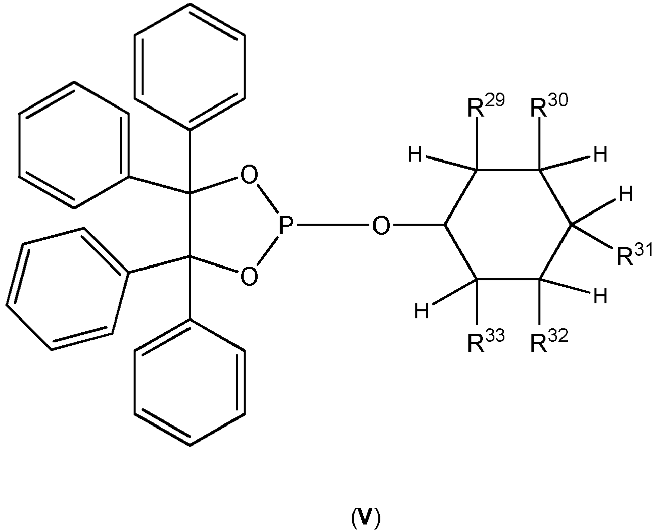 wobei
R¹, R², R³, R⁴, R⁵ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁶, R⁷, R⁸, R⁹, R¹⁰, R¹¹, D¹², R¹³, R¹⁴, R¹⁵, R¹⁶, R¹⁷, R¹⁸, R¹⁹, R²⁰, R²¹ R²², R²³, R²⁴, R²⁵, R²³, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
R²⁹, R³⁰, R³¹, R³², R³³ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl,-CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen folgendermaßen substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl:
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂;
und R¹ und R⁵ nicht für tert.-Butyl stehen,
und mindestens einer der Reste R¹, R², R³, R⁴, R⁵ nicht für -H steht,
und für den Fall, dass einer der Reste R¹, R², R³, R⁴, R⁵ für Phenyl steht, mindestens einer der vier verbleibenden Reste nicht für -H steht.

2. Verbindung nach Anspruch 1,
wobei R¹, R², R³, R⁴, R⁵ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

3. Verbindung nach einem der Ansprüche 1 oder 2,
wobei R⁶, R⁷, R⁸, R⁹, R¹⁰, R¹¹, R¹² ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

4. Verbindung nach einem der Ansprüche 1 bis 3,
wobei R¹³, R¹⁴, R¹⁵, R¹⁶, R¹⁷, R¹⁸, R¹⁹ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

5. Verbindung nach einem der Ansprüche 1 bis 4,
wobei R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

6. Verbindung nach einem der Ansprüche 1 bis 5,
wobei R²⁹, R³⁰, R³¹, R³², R³³ ausgewählt sind aus:
-H, -(C₁-C₁₂)-Alkyl, -O-(C₁-C₁₂)-Alkyl, -O-(C₆-C₂₀)-Aryl.

7. Verbindung nach einem der Ansprüche 1 bis 6,
wobei R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

8. Verbindung nach einem der Ansprüche 1 bis 7,
wobei die Verbindung die allgemeinen Strukturen **I** aufweist.

9. Verbindung nach einem der Ansprüche 1 bis 7,
wobei die Verbindung die allgemeinen Strukturen **II** aufweist.

10. Verbindung nach einem der Ansprüche 1 bis 7,
wobei die Verbindung die allgemeinen Strukturen **III** aufweist.

11. Verbindung nach einem der Ansprüche 1 bis 7,
wobei die Verbindung die allgemeinen Strukturen **IV** aufweist.

12. Verbindung nach einem der Ansprüche 1 bis 7,
wobei die Verbindung die allgemeinen Strukturen **V** aufweist.

13. Komplex umfassend:
- eine Verbindung nach einem der Ansprüche 1 bis 12,
- ein Metallatom ausgewählt aus: Rh, Ru, Co, Ir.

14. Verwendung einer Verbindung nach einem der Ansprüche 1 bis 12,
zur Katalyse einer Hydroformylierungsreaktion.

15. Verfahren umfassend die Verfahrensschritte:
a) Vorlegen eines Olefins,
b) Zugabe eines Komplexes nach Anspruch 13,
oder einer Verbindung nach einem der Ansprüche 1 bis 12 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having one of the general structures **I** to **V:** 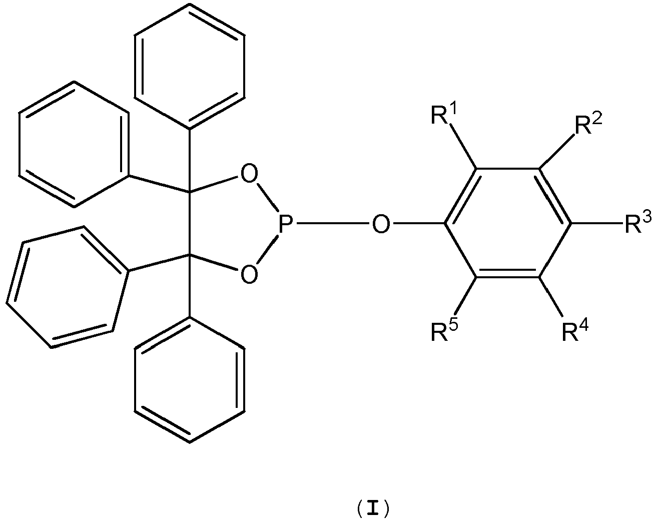 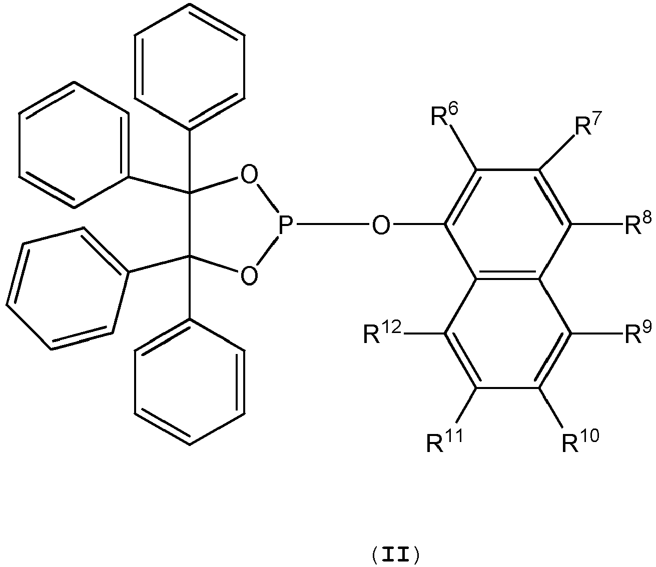 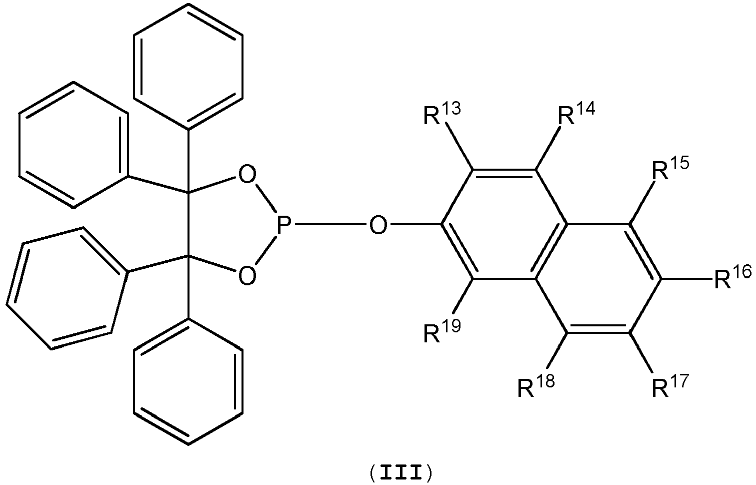 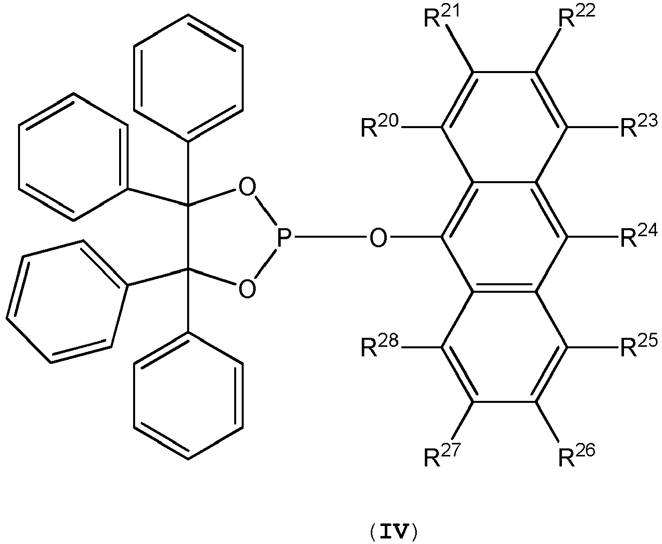 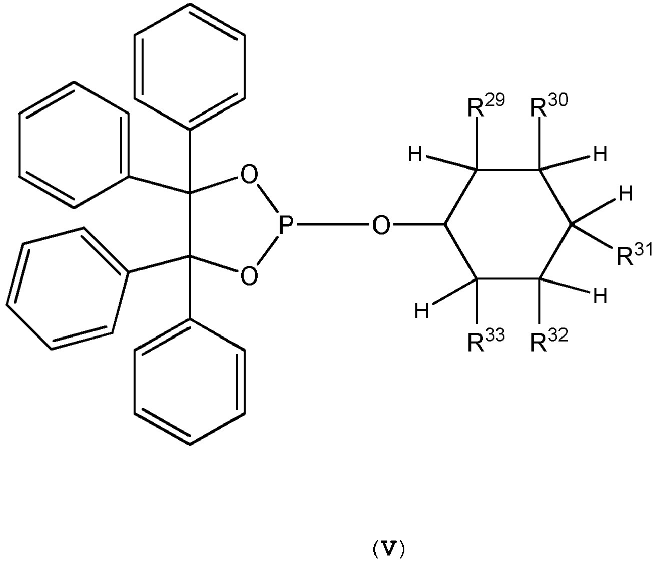 where
R¹, R², R³, R⁴, R⁵ are selected from:
-H, - (C₁-C₁₂)-alkyl, -O-(C₁-C₁₂)-alkyl, -O-(C₆-C₂₀)-aryl, - (C₆-C₂₀)-aryl, -S-alkyl, -S-aryl, halogen, -COO- (C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)- alkyl]₂;
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂) - alkyl]₂;
R²⁹, R³⁰, R³¹, R³², R³³ are selected from:
-H, -(C₁-C₁₂)-alkyl, -O-(C₁-C₁₂)-alkyl, -O-(C₆-C₂₀)-aryl, - (C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀) -aryl, -COOH, -OH, -SO₃H, -NH₂, -N[(C₁-C₁₂)- alkyl]₂;
where the alkyl and aryl groups mentioned may be substituted as follows:
substituted -(C₁-C₁₂)-alkyl groups and substituted -(C₁-C₁₂)-alkoxy groups may have one or more substituents, depending on their chain length; the substituents are each independently selected from -(C₃-C₁₂)-cycloalkyl, - (C₃-C₁₂)-heterocycloalkyl, -(C₆-C₂₀)-aryl, fluorine, chlorine, cyano, formyl, acyl and alkoxycarbonyl;
substituted -(C₆-C₂₀)-aryl groups and -(C₆-C₂₀)-aryl-(C₆-C₂₀)-aryl groups may have one or more substituents, depending on the ring size; these substituents are each independently selected from -H, -(C₁-C₁₂)-alkyl, -O-(C₆-C₂₀)-aryl, -(C₆-C₂₀)-aryl, -halogen -COO-(C₁-C₁₂)-alkyl, - CONH-(C₁-C₁₂)-alkyl, -(C₆-C₂₀)-aryl-CON[(C₁-C₁₂)-alkyl]₂, - CO-(C₁-C₁₂)-alkyl, -CO-(C₆-C₂₀)-aryl, -COOH, -OH, -SO₃H, - SO₃Na, -NO₂, -CN, -NH₂, -N[(C₁-C₁₂)-alkyl]₂;
and R¹ and R⁵ are not tert-butyl,
and at least one of the R¹, R², R³, R⁴, R⁵ radicals is not -H,
and if one of the R¹, R², R³, R⁴, R⁵ radicals is phenyl, at least one of the four remaining radicals is not -H.

2. Compound according to Claim 1,
where R¹, R², R³, R⁴, R⁵ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl.

3. Compound according to either of Claims 1 and 2, where R⁶, R⁷, R⁸, R⁹, R¹⁰, R¹¹, R¹² are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl.

4. Compound according to any of Claims 1 to 3,
where R¹³, R¹⁴, R¹⁵, R¹⁶, R¹⁷, R¹⁸, R¹⁹ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl.

5. Compound according to any of Claims 1 to 4,
where R²⁰, R²¹, R²², R²³, R²⁴, R²⁵, R²⁶, R²⁷, R²⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl.

6. Compound according to any of Claims 1 to 5,
where R²⁹, R³⁰, R³¹, R³², R³³ are selected from:
-H, -(C₁-C₁₂)-alkyl, -O-(C₁-C₁₂)-alkyl, -O-(C₆-C₂₀)-aryl.

7. Compound according to any of Claims 1 to 6,
where R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ R²², R²³, R²⁴, R²⁵, R²⁶, R²⁷, R²⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl.

8. Compound according to any of Claims 1 to 7,
where the compound has the general structure **I.**

9. Compound according to any of Claims 1 to 7,
where the compound has the general structure **II.**

10. Compound according to any of Claims 1 to 7,
where the compound has the general structure **III.**

11. Compound according to any of Claims 1 to 7,
where the compound has the general structure **IV.**

12. Compound according to any of Claims 1 to 7,
where the compound has the general structure **V.**

13. Complex comprising:
- a compound according to any of Claims 1 to 12,
- a metal atom selected from: Rh, Ru, Co, Ir.

14. Use of a compound according to any of Claims 1 to 12
for catalysing a hydroformylation reaction.

15. A process comprising the following process steps:
a) initially charging an olefin,
b) adding a complex according to Claim 13,
or a compound according to any of Claims 1 to 12 and a substance having a metal atom selected from: Rh, Ru, Co, Ir,
c) feeding in H₂ and CO,
d) heating the reaction mixture, with conversion of the olefin to an aldehyde.

## Revendications

1. Composé, qui présente une des structures générales I à V : 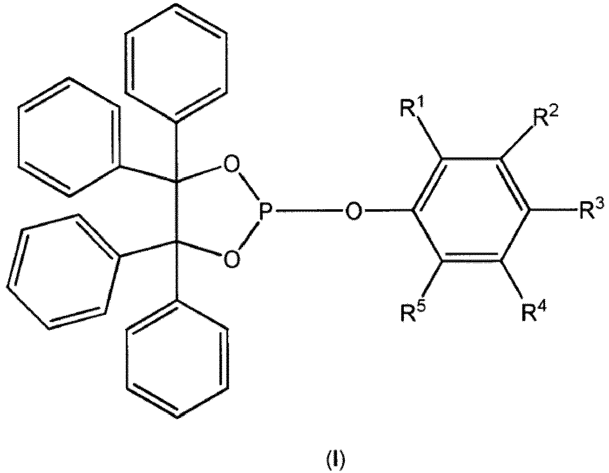 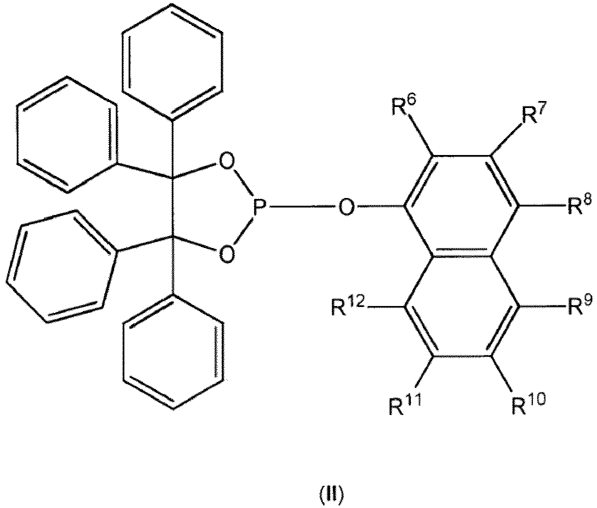 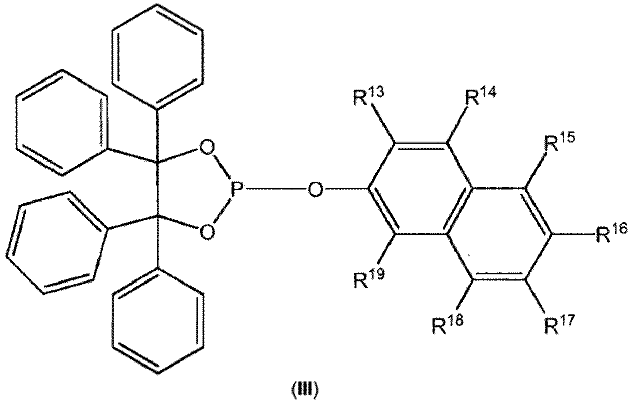 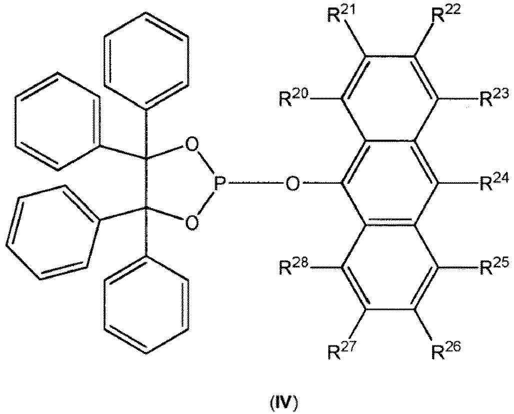 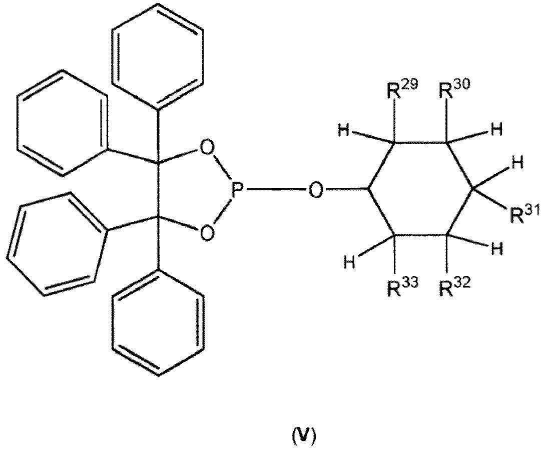 dans lesquelles
R¹, R², R³, R⁴, R⁵ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle, halogène, -COO-alkyle en (C₁-C₁₂), -CONH-alkyle en (C₁-C₁₂), -CO-alkyle en (C₁-C₁₂), -CO-aryle en (C₆-C₂₀), -COOH, -OH, -SO₃H, -CN, -NH₂, -N[alkyle en (C₁-C₁₂)]₂;
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle, halogène, -COO-alkyle en (C₁-C₁₂), -CONH-alkyle en (C₁-C₁₂), -CO-alkyle en (C₁-C₁₂), -CO-aryle en (C₆-C₂₀), -COOH, -OH, -SO₃H, -NH₂, -N[alkyle en (C₁-C₁₂)]₂;
R²⁹, R³⁰, R³¹, R³², R³³ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle, halogène, -COO-alkyle en (C₁-C₁₂), -CONH-alkyle en (C₁-C₁₂), -CO-alkyle en (C₁-C₁₂), -CO-aryle en (C₆-C₂₀), -COOH, -OH, -SO₃H, -NH₂, -N[alkyle en (C₁-C₁₂)]₂;
les groupes alkyle et aryle mentionnés pouvant être substitués de la manière suivante :
les groupes alkyle en (C₁-C₁₂) substitués et les groupes alcoxy en (C₁-C₁₂) substitués peuvent comprendre, en fonction de leur longueur de chaîne, un ou plusieurs substituants; les substituants sont choisis indépendamment les uns des autres parmi cycloalkyle en (C₃-C₁₂), hétérocycloalkyle en (C₃-C₁₂), aryle en (C₆-C₂₀), fluor, chlore, cyano, formyle, acyle ou alcoxycarbonyle ;
les groupes aryle en (C₆-C₂₀) et les groupes aryle en (C₆-C₂₀) -aryle en (C₆-C₂₀) substitués peuvent comprendre, en fonction de la taille du cycle, un ou plusieurs substituants; ces substituants sont choisis indépendamment les uns des autres parmi -H, -alkyle en (C₁-C₁₂), -O-aryle en (C₆-C₂₀), -aryle en (C₆-C₂₀), - halogène, -COO-alkyle en (C₁-C₁₂), -CONH-alkyle en (C₁-C₁₂), -aryle en (C₆-C₂₀) -CON[alkyle en (C₁-C₁₂)]₂, -CO-alkyle (C₁-C₁₂), -CO-aryle en (C₆-C₂₀), -COOH, -OH, -SO₃H; - SO₃Na, -NO₂, -CN, -NH₂, -N[alkyle en (C₁-C₁₂)]₂;
et R¹ et R⁵ ne représentent pas tert.-butyle,
et au moins un des radicaux R¹, R², R³, R⁴, R⁵ ne représente pas -H,
et lorsqu'un des radicaux R¹, R², R³, R⁴, R⁵ représente phényle, au moins un des quatre radicaux restants ne représente pas -H.

2. Composé selon la revendication 1, dans lequel R¹, R², R³, R⁴, R⁵ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle.

3. Composé selon l'une quelconque des revendications 1 ou 2, dans lequel R⁶, R⁷, R⁸, R⁹, R¹⁰, R¹¹, R¹² sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle.

4. Composé selon l'une quelconque des revendications 1 à 3, dans lequel R¹³, R¹⁴, R¹⁵, R¹⁶, R¹⁷, R¹⁸, R¹⁹ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle.

5. Composé selon l'une quelconque des revendications 1 à 4, dans lequel R²⁰, R²¹, R²², R²³, R²⁴, R²⁵, R²⁶, R²⁷, R²⁸ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle.

6. Composé selon l'une quelconque des revendications 1 à 5, dans lequel R²⁹, R³⁰, R³¹, R³², R³³ sont choisis parmi :
-H, -alkyle en (C₁-C₁₂), -O-alkyle en (C₁-C₁₂), -O-aryle en (C₆-C₂₀).

7. Composé selon l'une quelconque des revendications 1 à 6, dans lequel R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle.

8. Composé selon l'une quelconque des revendications 1 à 7, dans lequel le composé présente la structure générale I.

9. Composé selon l'une quelconque des revendications 1 à 7, dans lequel le composé présente la structure générale II.

10. Composé selon l'une quelconque des revendications 1 à 7, dans lequel le composé présente la structure générale III.

11. Composé selon l'une quelconque des revendications 1 à 7, dans lequel le composé présente la structure générale IV.

12. Composé selon l'une quelconque des revendications 1 à 7, dans lequel le composé présente la structure générale V.

13. Complexe, comprenant :
- un composé selon l'une quelconque des revendications 1 à 12,
- un atome métallique choisi parmi : Rh, Ru, Co, Ir.

14. Utilisation d'un composé selon l'une quelconque des revendications 1 à 12 pour la catalyse d'une réaction d'hydroformylation.

15. Procédé comprenant les étapes de procédé suivantes :
a) le chargement d'une oléfine,
b) l'ajout d'un complexe selon la revendication 13,
ou d'un composé selon l'une quelconque des revendications 1 à 12 et d'une substance qui comprend un atome métallique choisi parmi : Rh, Ru, Co, Ir,
c) l'introduction d'H₂ et de CO,
d) le chauffage du mélange réactionnel, l'oléfine étant transformée en un aldéhyde.
